# EUROPEAN PATENT APPLICATION

(11) **EP 3 604 341 A1**
(43) Date of publication of application: **05.02.2020**
(21) Application number: 18776444.4
(22) Date of filing: 02.03.2018
(51) Int. Cl.: C07K 17/00, B01D 15/38, B01J 20/281, C07K 1/22, C07K 14/315, C07K 19/00

(54) **AFFINITY DISSOCIATION MATRIX FOR IMMUNOGLOBULIN PURIFICATION USE**

(30) Priority: 31.03.2017 JP 2017071909
(71) Applicant: Kaneka Corporation, Osaka-shi, Osaka 530-8288 (JP)
(72) Inventor: YOSHIDA, Shinichi, Takasago-shi Hyogo 676-8688 (JP)
(74) Representative: Hoffmann Eitle
(86) International application number: PCT/JP2018/008051
(87) International publication number: WO 2018/180205

(57) **Abstract**

The objective of the present invention is to provide a novel affinity separation matrix which has a binding ability to an immunoglobulin and which is excellent in a chemical stability to an alkaline solution, and a method for producing an immunoglobulin by using the affinity separation matrix. The separation matrix according to the present invention is characterized in having a novel peptide as a ligand. The peptide has an amino acid sequence of a protein of which amino acid sequence identity to an immunoglobulin-binding domain of Protein G is high but of which difference of physical property and function from Protein G has been unclear.

## Description

### TECHNICAL FIELD

The present invention relates to an affinity separation matrix containing an immunoglobulin-binding peptide as a ligand of which chemical stability to an alkaline solution is improved, and a method for producing an antibody or an antibody fragment by using the affinity separation matrix.

### BACKGROUND ART

One of the examples of important functions of a protein includes an ability to specifically bind to a specific molecule. The ability plays an important role in an immunoreaction and signal transduction in a living body. A technology utilizing the ability for purifying a useful substance has been actively developed. One of the examples of a protein which is actually utilized industrially includes Protein A affinity separation matrix (Non-patent documents 1 and 2). The Protein A affinity separation matrix is used for capturing an antibody drug to be purified with high purity at one time from a culture product of an animal cell. Hereinafter, Protein A is abbreviated as "SpA" in some cases. An antibody drug which has been developed is mainly a monoclonal antibody, and a monoclonal antibody has been produced on a large scale by using recombinant cell cultivation technology. A "monoclonal antibody" means an antibody obtained from a clone derived from a single antibody-producing cell. Most of antibody drugs launched presently are classified into an immunoglobulin G (IgG) subclass in terms of a molecular structure.

A protein referred to as Protein G found in Streptococcus sp. classified into Group G can also bind to IgG. Hereinafter, Protein G is abbreviated as "SpG" in some cases. An SpG affinity separation matrix product on which SpG is immobilized as a ligand has been commercially available (product name: "Protein-G Sepharose 4 Fast Flow" manufactured by GE Healthcare, Patent Document 1). SpG is characterized in strongly binding to an Fc region of an IgG and also strongly binding to a broader kind of an animal IgG than SpA. It is further known that SpG weakly binds to a Fab region (Non-patent documents 3 and 4). The ability of SpG to bind to a Fab region is tried to be improved (Patent documents 2 to 4, Non-patent document 5).

An SpA affinity separation matrix is more widely used for purifying an antibody drug in comparison with SpG. It is considered to be one of the reasons that SpA has higher stability to an alkaline solution than SpG (Non-patent document 1). When the stability to an alkali is high, such an affinity separation matrix can be regenerated to be repeatedly used by being washed with an aqueous solution of sodium hydroxide. A sodium hydroxide aqueous solution is inexpensive and highly effective in cleaning and sterilizing. The example of a method for enhancing the stability of a protein ligand of an affinity separation matrix to an alkali includes a protein engineering technique such as an introduction of an amino acid mutation. Specifically, a substitution of an asparagine residue or a glycine residue after an asparagine residue has an effect on the improvement of a chemical stability, since an asparagine residue is susceptible to deamination reaction under an alkaline condition (Non-patent document 1). On the one hand, all of such asparagine residue mutations in SpA do not necessarily show the improvement effect (Non-patent documents 1 and 6). A similar study to introduce a mutation in an asparagine residue is also made for SpG (Patent document 5, Non-patent documents 7 and 8); but there is a room for improvement, since not all mutations are effective as the case of SpA and a stability to an alkali comparable to that of SpA has not been achieved.

### PRIOR ART DOCUMENT

### PATENT DOCUMENT

Patent Document 1: JP S63-503032 A
Patent Document 2: JP 2009-195184 A
Patent Document 3: WO 2015/030094
Patent Document 4: WO 2016/061427
Patent Document 5: JP 2016-079153 A

### NON-PATENT DOCUMENT

Non-patent Document 1: Hober S. et al., J. Chromatogr. B, 2007, vol. 848, pp. 40-47
Non-patent Document 2: Shukla A. A. et al. , Trends Biotechnol., 2010, vol. 28, pp. 253-261
Non-patent Document 3: Bouvet P. J. et al., Int. J. Immunopharmac., 1994, vol. 16, pp. 419-424
Non-patent Document 4: Derrick J. P. et al., Nature, 1992, vol. 359, pp. 752-754
Non-patent Document 5: Bailey L. J. et al., J. Immunol. Methods, 2014, vol. 415, pp. 24-30
Non-patent Document 6: Linhult M. et al., PROTEINS, 2004, vol. 55, pp. 407-416
Non-patent Document 7: Gulich S. et al., Protein Eng., 2002, vol. 15, pp. 835-842
Non-patent Document 8 : Palmer B. et al., J. Biotechnol., 2008, vol. 134, pp. 222-230

### DISCLOSURE OF THE INVENTION

### PROBLEMS TO BE SOLVED BY THE INVENTION

The objective of the present invention is to provide an affinity separation matrix which can bind to an Fc region and a Fab region of an immunoglobulin and which is excellent in a chemical stability to an alkaline solution, and a method for producing an antibody or an antibody fragment by using the affinity separation matrix.

### MEANS FOR SOLVING THE PROBLEMS

The inventor of the present invention completed the present invention by evaluating a physical property and a function of an amino acid sequence of a protein of which amino acid sequence identity to an amino acid sequence of an immunoglobulin-binding domain of SpG is high but of which physical property and function are unclear (Jonsson H, Lindmark H, Guss B., Infect Immun., 1995, vol. 8, 2968-75) by using a protein engineering method and a genetic engineering method in order to solve the above-described problem.

Hereinafter, the present invention is described.
[1] An affinity separation matrix,
   comprising an immunoglobulin-binding peptide and a water-insoluble carrier,
   wherein the immunoglobulin-binding peptide comprises an amino acid sequence of SEQ ID NO: 1 or an amino acid sequence with a sequence identity of 94% or more to the amino acid sequence of SEQ ID NO: 1, and
   the immunoglobulin-binding peptide is immobilized on the water-insoluble carrier as a ligand.
[2] The affinity separation matrix according to the above [1], wherein the amino acid sequence of SEQ ID NO: 1 is any one of amino acid sequences of SEQ ID NOs: 2 to 16.
[3] The affinity separation matrix according to the above [1], wherein the immunoglobulin-binding peptide comprises an amino acid sequence of SEQ ID NO: 1 with deletion, substitution and/or addition of one or more amino acids at N-terminal and/or C-terminal.
[4] The affinity separation matrix according to any one of the above [1] to [3], wherein an immunoglobulin-binding peptide multimer comprising two or more of the immunoglobulin-binding peptides connected to one another is immobilized on the water-insoluble carrier as the ligand.
[5] A method for producing a protein comprising an Fc region and/or a Fab region of an immunoglobulin, comprising:
   contacting a liquid sample comprising the protein with the affinity separation matrix according to any one of the above [1] to [4], and
   separating the protein adsorbed on the affinity separation matrix from the affinity separation matrix.

### EFFECT OF THE INVENTION

The immunoglobulin-binding activity of the chromatography carrier for affinity purification on which the peptide according to the present invention is immobilized is less likely to be decreased due to a damage by an alkali treatment. It is therefore possible to wash the carrier by using a high concentration sodium hydroxide aqueous solution for a long time for repeated use.

### BRIEF DESCRIPTION OF THE DRAWINGS

Figure 1 are graphs to demonstrate binding parameters of the immunoglobulin-binding peptides of Example 2 according to the present invention to an IgG.
Figure 2 are graphs to demonstrate binding parameters of the immunoglobulin-binding peptides of Example 2 according to the present invention to a Fab.
Figure 3 are graphs to demonstrate an alkali resistance of the immunoglobulin-binding peptides of Example 3 according to the present invention.

### MODE FOR CARRYING OUT THE INVENTION

The affinity separation matrix is characterized in comprising an immunoglobulin-binding peptide and a water-insoluble carrier, wherein the immunoglobulin-binding peptide comprises an amino acid sequence of SEQ ID NO: 1 or an amino acid sequence with a sequence identity of 94% or more to the amino acid sequence of SEQ ID NO: 1, and the immunoglobulin-binding peptide is immobilized on the water-insoluble carrier as a ligand. The sequence of SEQ ID NO: 1 is shown as follows. 
SEQ ID NO: 1: Xaa01-Xaa02-Tyr-Lys-Leu-Xaa06-Val-Lys-Gly-Xaa10-
Thr-Xaa12-Xaa13-Gly-Xaa15-Thr-Xaa17-Thr-Xaa19-Ala-Xaa21-Asp-Xaa23-
Xaa24-Xaa25-Ala-Glu-Xaa28-Xaa29-Xaa30-Xaa31-Xaa32-Xaa33-Ala-Xaa35-
Xaa36-Asn-Xaa38-Xaa39-Xaa40-Gly-Xaa42-Trp-Xaa44-Tyr-Asp-Xaa47-Ala-
Thr-Lys-Thr-Phe-Thr-Val-Thr-Glu
 Xaa⁰¹ = Asp/Thr, Xaa⁰² = Thr/Ser, Xaa⁰⁶ = Val/Ile, Xaa¹⁰ = Ala/Val/Asn, Xaa¹² = Phe/Leu, Xaa¹³ = Ser/Thr, Xaa¹⁵ = Glu/Tyr, Xaa¹⁷ = Thr/Ala, Xaa¹⁹ = Lys/Ile, Xaa²¹ = Val/Ala, Xaa²³ = Ala/Thr, Xaa²⁴ = Ala/Glu, Xaa²⁵ = Thr/Val, Xaa²⁸ = Lys/Gln, Xaa²⁹ = Ala/Glu/Thr, Xaa^{3C} = Phe/Leu, Xaa³¹ = Lys/Arg, Xaa³² = Gln/Asp, Xaa³³ = Tyr/Phe, Xaa³⁵ = Thr/Asn/Phe, Xaa³⁶ = Ala/Asp/Glu/Lys, Xaa³⁸ = Asn/Gly, Xaa³⁹ = Val/Thr, Xaa⁴⁰ = Asp/Tyr/Thr, Xaa⁴² = Glu/Val, Xaa⁴⁴ = Ser/Ala, Xaa⁴⁷ = Asp/Ala/Thr

In the above definition, "/" represents "or". The above-described sequence identity is more preferably not less than 95% or not less than 96%, even more preferably not less than 98% or not less than 99%, and even more preferably not less than 99.5% or not less than 99.8%.

An "immunoglobulin (IgG)" is a glycoprotein produced by a B cell of a lymphocyte and has a function to recognize a molecule to be bound, such as a specific protein. An immunoglobulin has not only a function to specifically bind to a specific molecule referred to as antigen but also a function to detoxify and remove an antigen-containing factor in cooperation with other biological molecule or cell. An immunoglobulin is generally referred to as "antibody", and the name is inspired by such functions. All of immunoglobulins basically have the same molecular structure. The basic structure of an immunoglobulin is a γ-shaped four-chain structure consisting of two light chains and two heavy chains of polypeptides. A light chain (L chain) is classified into two types of λ chain and κ chain, and all of immunoglobulins have either of the types. A heavy chain (H chain) is classified into five types of γ chain, µ chain, α chain, δ chain and ε chain, and the kind of an immunoglobulin, i.e. isotype, is different depending on a heavy chain. An immunoglobulin G (IgG) is a monomer immunoglobulin, is composed of two heavy chains (γ chains) and two light chains, and has two antigen-binding sites.

A lower half vertical part in the "Y" shape of an immunoglobulin is referred to as an "Fc region", and an upper half "V" shaped part is referred to as a "Fab region". An Fc region has an effector function to initiate a reaction after an antibody binds to an antigen, and a Fab region has a function to bind to an antigen. The Fab region and Fc region of a heavy chain are bound to each other through a hinge part. Papain, which is a proteolytic enzyme and which is contained in papaya, decomposes a hinge part to cut into two Fab regions and one Fc region. The domain part close to the edge of the "Y" shape in a Fab region is referred to as a "variable region (V region)", since there are various changes in the amino acid sequence in order to bind to various antigens. A variable region of a light chain is referred to as a "VL region", and a variable region of a heavy chain is referred to as a "VH region". The other part in a Fab region except for a V region and an Fc region are referred to as a "constant region (C region)", since there is relatively less change. The constant region of a light chain is referred to as a "CL region", and the constant region of a heavy chain is referred to as a "CH region". A CH region is further classified into three regions of CH1 to CH3. A Fab region of a heavy chain is composed of a VH region and CH1, and an Fc region of a heavy chain is composed of CH2 and CH3. There is a hinge part between CH1 and CH2.

The term "peptide" in the present invention means any molecules having a polypeptide structure. In the range of the "peptide", not only a so-called protein but also a fragmented peptide and a peptide to which other peptide is bound through a peptide bond are included. The term "domain" means a unit of higher-order structure of a protein. A domain is composed of from dozens to hundreds of amino acid residues and means a protein unit which can sufficiently serve some kind of a physicochemical or biochemical function. The phrase, a peptide "has a (specific) amino acid sequence", in the present invention means that the specific amino acid sequence is contained in the amino acid sequence of the peptide and the function of the peptide is maintained. The sequence of a peptide other than a specific amino acid sequence in the peptide is exemplified by histidine tag, a linker sequence for immobilization, and a crosslinking structure such as -S-S- bond.

Even when, for example, a peptide is added to N-terminal, a skilled person can easily identify a position of each residue of SEQ ID NO: 1 on the basis of the sequence identity. For example, the position can be identified by the alignment function of gene information processing software: GENETYX (https://www.genetyx.co.jp/). The sequence identity needed for identifying the position is preferably 94% or more, more preferably not less than 95% or not less than 96%, even more preferably not less than 98% or not less than 99%, and even more preferably not less than 99.5% or not less than 99.8%.

The term "ligand" means a substance and a functional group to selectively bind to and adsorb a target molecule from an aggregate of molecules on the basis of a specific affinity between molecules, such as binding between an antigen and an antibody, and means the peptide which specifically binds to an immunoglobulin in the present invention. The term "affinity ligand" also means a "ligand" in the present invention.

A water-insoluble carrier usable in the present invention is exemplified by an inorganic carrier such as glass beads and silica gel; an organic carrier; and a composite carrier obtained from the combination of the above carriers, such as an organic-organic composite carrier and an organic-inorganic composite carrier. The example of an organic carrier includes a carrier composed of a synthetic polymer such as cross-linked polyvinyl alcohol, cross-linked polyacrylate, cross-linked polyacrylamide and cross-linked polystyrene; a carrier composed of a polysaccharide such as crystalline cellulose, cross-linked cellulose, cross-linked agarose and cross-linked dextran. The commercial product thereof is exemplified by porous cellulose gel GCL2000, Sephacryl S-1000 prepared by crosslinking allyl dextran and methylene bisacrylamide through a covalent bond, an acrylate carrier Toyopearl, a cross-linked agarose carrier Sepharose CL4B, and a cross-linked cellulose carrier Cellufine. It should be noted that the water-insoluble carrier usable in the present invention is not restricted to the carriers exemplified as the above.

It is preferred that the water-insoluble carrier usable in the present invention has large surface area and that the carrier is porous with a large number of fine pores having a suitable size in terms of a purpose and method for using the affinity separation matrix according to the present invention. The carrier can have any form such as beads, monolith, fiber and film, and any form can be selected. A film may be a hollow fiber.

With respect to a method for immobilizing the ligand, for example, the ligand can be bound to a carrier by a conventional coupling method utilizing an amino group, a carboxy group or a thiol group of the ligand. Such a coupling method is exemplified by an immobilization method including activation of a carrier by a reaction with cyanogen bromide, epichlorohydrin, diglycidyl ether, tosyl chloride, tresyl chloride, hydrazine, sodium periodate or the like, or introduction of a reactive functional group on the carrier surface, and the coupling reaction between the resulting carrier and a compound to be immobilized as a ligand; and an immobilization method by condensation and crosslinking which method includes adding a condensation reagent such as carbodiimide or a reagent having a plurality of functional groups in the molecule, such as glutaraldehyde, into a mixture containing a carrier and a compound to be immobilized as a ligand.

A spacer molecule composed of a plurality of atoms may be introduced between the ligand and carrier. Alternatively, the ligand may be directly immobilized on the carrier. Accordingly, the immunoglobulin-binding peptide usable in the affinity separation matrix according to the present invention may be chemically modified for immobilization, or may have an amino acid residue useful for immobilization. Such an amino acid useful for immobilization is exemplified by an amino acid having a functional group useful for a chemical reaction for immobilization in a side chain, and specifically exemplified by Lys having an amino group in the side chain and Cys having a thiol group in the side chain. Since the immunoglobulin-binding ability of the peptide according to the present invention is principally maintained in a matrix prepared by immobilizing the peptide as a ligand in the present invention, any modification and change for immobilization are included in the range of the present invention.

It is more preferred that the affinity separation matrix according to the present invention is prepared by immobilizing the immunoglobulin-binding peptide having the amino acid sequences of SEQ ID NOs: 2 to 16 or an amino acid sequence with a sequence identity of 94% or more to the amino acid sequences on a water-insoluble carrier as a ligand. The sequence identity is more preferably not less than 95% or not less than 96%, even more preferably not less than 98% or not less than 99%, and even more preferably not less than 99.5% or not less than 99.8%.

It is more preferred that the affinity separation matrix to purify an altered immunoglobulin which does not contain an Fc region and which contains a Fab region only is prepared by immobilizing the immunoglobulin-binding peptide having the amino acid sequences of SEQ ID NOs: 8 to 13 or an amino acid sequence with a sequence identity of 94% or more to the amino acid sequences on a water-insoluble carrier as a ligand. The sequence identity is more preferably not less than 95% or not less than 96%, even more preferably not less than 98% or not less than 99%, and even more preferably not less than 99.5% or not less than 99.8%.

The embodiment that the immunoglobulin-binding peptide of the affinity separation matrix according to the present invention has an amino acid sequence in which one or more amino acids are deleted, substituted and/or added is also included in the present invention.

The range of the "one or more" is not particularly restricted as long as the immunoglobulin-binding peptide with deletion or the like has a strong binding ability to an immunoglobulin. The above-described range of the "one or more" is exemplified by 30 or less, preferably 20 or less, more preferably 10 or less, even more preferably 7 or less, even more preferably 5 or less, and particularly preferably 3 or less, 1 or 2, or 1.

The position of the deletion, substitution and/or addition of an amino acid residue is exemplified by N-terminal and/or C-terminal. The terminal positions are particularly preferred as the position of the deletion and/or addition. As the embodiment of an amino acid sequence to be added, it is exemplified that an amino acid sequence containing Lys or Cys, which are useful for immobilizing the peptide on a matrix, is added to the C-terminal side.

The immunoglobulin-binding peptide with the above-described sequence identity and the above-described mutation, i.e. deletion, substitution and/or addition, is also excellent in a chemical stability. Specifically, a chemical stability of the immunoglobulin-binding peptide with the sequence identity and the mutation to an alkaline aqueous solution is preferably superior to that of wild SpG, and is more preferably equivalent or superior to that of the immunoglobulin-binding peptide having the amino acid sequence of SEQ ID NO: 1. An binding activity of the immunoglobulin-binding peptide with the sequence identity and the mutation to an Fc region and/or a Fab region is preferably superior to that of wild SpG, and is more preferably equivalent or superior to that of the immunoglobulin-binding peptide having the amino acid sequence of SEQ ID NO: 1.

Even when the number of amino acids is changed by the above-described deletion or addition, the position of an amino acid residue after introducing the mutation which position corresponds to the position of an amino acid residue before introducing the mutation can be easily identified by alignment analysis between the amino acid sequences before and after introducing the mutation. The means of such an alignment analysis is widely known by a person skilled in the art.

The affinity separation matrix obtained by the present invention is characterized in having high chemical stability to an alkaline aqueous solution. In other words, even when the affinity separation matrix is treated by an alkaline aqueous solution, the matrix is less damaged by an alkali and maintains a high level immunoglobulin-binding performance.

The "alkaline aqueous solution" shows an alkalinity to the extent that the purpose of cleaning or sterilization can be achieved. More specifically, a sodium hydroxide aqueous solution of 0.01 M or more and 1.0 M or less or 0.01 N or more and 1.0 N or less can be used, but the alkaline aqueous solution is not restricted thereto. With respect to sodium hydroxide as an example, the lower limit of the concentration thereof is preferably 10 mM, more preferably 15 mM, more preferably 20 mM, and even more preferably 25 mM. On the one hand, the upper limit of a sodium hydroxide concentration is preferably 1.0 M, more preferably 0.5 M, more preferably 0.3 M, more preferably 0.2 M, and even more preferably 0.1 M. The alkaline aqueous solution is not necessarily a sodium hydroxide aqueous solution. A pH thereof is preferably 12 or more and 14 or less. With respect to the lower limit of the pH, 12.0 or more is preferred, and 12.5 or more is more preferred. With respect to the upper limit of the pH, 14 or less is preferred, 13.5 or less is more preferred, and 13.0 or less is even more preferred.

The term "chemical stability" means a property to maintain a function of the peptide against a chemical modification such as a chemical change of an amino acid residue and a chemical denaturation such as a transition and a breakage of an amide bond. The function to be maintained of the peptide in the present invention means a binding activity to an immunoglobulin. The term "binding activity to an immunoglobulin" in the present invention means a ratio of the polypeptide which retains an affinity for an immunoglobulin without chemical denaturation. Thus, when the "chemical stability" is higher, a degree of decrease of a binding activity to an immunoglobulin is smaller even after an immersion treatment in an alkaline aqueous solution. The term "alkali resistance" in this disclosure has the same meaning as "chemical stability under an alkaline condition".

The time to immerse the peptide in an alkali is not particularly restricted, since a damage to the peptide is critically different depending on an alkaline concentration and a temperature during the immersion. For example, in the case where a concentration of sodium hydroxide is 15 mM and a temperature during the immersion is room temperature, the lower limit time for the immersion in an alkali is preferably 30 minutes, more preferably 1 hour, more preferably 2 hours, more preferably 4 hours, more preferably 10 hour, and more preferably 20 hours, but is not particularly restricted thereto.

A binding activity to an antibody or an antibody fragment can be evaluated by a biosensor such as Biacore system (GE Healthcare) using a surface plasmon resonance principle and Octet (Pall ForteBio) using bioLayer interferometry, but the means is not restricted thereto. With respect to a condition for measuring a binding activity, a binding signal at the time of binding to an antibody or a fragment thereof may be detected. With respect to a measuring condition, the temperature is preferably adjusted to a constant temperature of 20°C or higher and 40°C or lower. The pH for evaluating a binding condition is preferably adjusted to a neutral condition of 5 or more and 8 or less. The example of a neutral buffer component includes phosphate, tris and bis-tris, but is not restricted thereto. A sodium chloride concentration in a buffer is not particularly restricted and is preferably 0 M or more and 0.15 M or less.

For example, an association constant (K_{A}) and a dissociation constant (K_{D}) can be used as a binding parameter to demonstrate the binding to an antibody or an antibody fragment (Nagata et al., "Real-Time Analysis Experimental Method for Interaction Between Biological Substances", Springer-Verlag Tokyo, 1998, p. 41). For example, an association constant between the present invention peptide and an antibody or an antibody fragment can be measured by immobilizing a human IgG or a fragment thereof on a biosensor and adding each altered domain into a flow channel under a condition of the temperature of 25°C and pH 7.4 in Octet system. An association constant (K_{A}) of the immunoglobulin-binding peptide usable in the affinity separation matrix of the present invention to a human immunoglobulin is preferably 1 x 10⁵ (M⁻¹) or more, and more preferably 1 x 10⁶ (M⁻¹) or more, but is not restricted thereto, since an association constant is also changed depending on a kind of an immunoglobulin and a domain number of an immunoglobulin-binding peptide.

When a residual binding activity after an alkali treatment is evaluated, K_{A} and K_{D} are inappropriate as a binding parameter, since when a ratio of a molecule which can bind to an antibody or an antibody fragment is changed by an alkali treatment but a binding ability of one peptide molecule to an antibody or an antibody fragment is not changed, K_{A} and K_{D} are not changed as a parameter. When a residual binding activity of the peptide is evaluated, for example, a binding signal value or a theoretical maximum binding capacity (Rₘₐₓ) is preferably used but the standard is not restricted thereto. The binding signal value and Rₘₐₓ are measured to an antibody or an antibody fragment immobilized on a sensor chip in the same concentration before and after the peptide is subjected to an alkali treatment. The binding signal value and Rₘₐₓ are binding parameters of which unit is a binding response (nm). For example, binding signal values to an antibody or an antibody fragment of the same concentration before and after a chip on which the peptide is immobilized is subjected to an alkali treatment may be compared.

Since a residual binding activity relates to a comparison before and after an alkali treatment, a residual binding activity can be basically shown as a ratio (percentage) calculated by dividing a binding activity after the alkali treatment as a numerator by a binding activity before the alkali treatment as a denominator. The value is not particularly restricted as long as the value is higher than that of a peptide into which the mutation of the present invention is not introduced and which is treated by an alkali in the same condition, and the ratio is preferably 10% or more, more preferably 20% or more, even more preferably 30% or more, even more preferably 40% or more, and even more preferably 50% or more.

As one of the embodiments, the immunoglobulin-binding peptide usable in the affinity separation matrix of the present invention may be a multimer of 2 or more monomers or single domains of the immunoglobulin-binding peptide connected to one another. The number of the monomers or single domains is preferably 3 or more, more preferably 4 or more, and even more preferably 5 or more. The upper limit of the number of connected domains may be, for example, 10, preferably 8, and more preferably 6. Such a multimer may be a homomultimer in which one kind of the immunoglobulin-binding peptides are connected, such as homodimer and homotrimer, or a heteromultimer in which two or more kinds of the immunoglobulin-binding peptides are connected, such as heterodimer and heterotrimer.

A method for connecting the immunoglobulin-binding peptides is exemplified by a connecting method through one or more amino acid residues and a method for directly connecting the monomer peptides without using an amino acid residue; however, is not restricted to the exemplified methods. The number of the amino acid residue for connection is not particularly restricted, and is preferably 1 residue or more and 20 residues or less, more preferably 15 residues or less, even more preferably 10 residues or less, even more preferably 5 residues or less, and even more preferably 2 residues or less. When the peptides are connected, it is preferred that the amino acid residue for connection does not destabilize a three dimensional structure of the monomer immunoglobulin-binding peptide.

The immunoglobulin-binding peptide may be bound by other peptide and compound. For example, the affinity separation matrix containing a fusion peptide prepared by fusing the immunoglobulin-binding peptide or the multimer of the two or more peptides connected to one another with other peptide having different function as one structural component is exemplified as one embodiment. The example of such a fusion peptide includes a peptide fused with albumin or GST, i.e. glutathione S-transferase, but is not restricted to the examples. In addition, peptides fused with a nucleic acid such as DNA aptamer, a drug such as antibiotic or a polymer such as PEG, i.e. polyethylene glycol, are also included in the range of the present invention as long as such a fusion peptide utilizes the utility of the peptide usable in the affinity separation matrix of the present invention.

It becomes possible by using the affinity separation matrix of the present invention that a protein containing an Fc region and/or a Fab region of an immunoglobulin is purified in accordance with affinity column chromatography purification method. A protein can be purified by a procedure in accordance with a method for purifying an immunoglobulin by affinity column chromatography (Non-Patent Document 1). Specifically, after a buffer which contains the protein and of which pH is approximately neutral is prepared, the solution is contacted with the affinity separation matrix of the present invention, for example, the solution is allowed to pass through an affinity column filled with the affinity separation matrix, so that the protein is adsorbed. Then, an appropriate amount of a pure buffer is allowed to pass through the affinity column to wash the inside of the column. At the time, the target protein is still adsorbed on the affinity separation matrix of the present invention in the column. The affinity separation matrix on which the present invention peptide is immobilized as a ligand is excellent in the absorption and retention performance for the target protein from the step to add a sample through the step to wash the matrix. Then, an acid buffer of which pH is appropriately adjusted is allowed to pass through the column to elute the target protein. As a result, purification with high purity can be achieved. Into the acid buffer for elution, a substance for promoting dissociation from the matrix may be added. The above-described target protein may be an immunoglobulin itself or an antibody fragment such as an Fc fragment and a Fab fragment.

The affinity separation matrix according to the present invention can be reused by allowing an adequate strong acidic or strong alkaline pure buffer which does not completely impair the function of the ligand compound and the base material of the carrier to pass through the matrix for washing. An adequate modifying agent or an organic solvent may be contained in the buffer for regeneration. It is preferred to reuse the affinity separation matrix of the present invention by passing a pure strong alkaline buffer for washing, since the affinity separation matrix is particularly excellent in the chemical stability to an alkaline aqueous solution. The timing for regeneration by a pure strong alkaline buffer needs not to be every time after use and for example, may be once every five times or once every ten times.

The DNA encoding the peptide usable in the affinity separation matrix of the present invention may be any DNA as long as the amino acid sequence produced from a translation of the base sequence of the DNA constitutes the peptide. Such a base sequence can be obtained by a generally used known methods such as polymerase chain reaction (hereinafter, abbreviated as "PCR") method. Alternatively, the DNA can be synthesized by publicly-known chemical synthesis method or is available from DNA library. A codon in the base sequence may be substituted by a degenerate codon, and the base sequence is not necessarily the same as the original base sequence as long as the translated amino acid is the same as that encoded by the original base sequence. It is possible to obtain a recombinant DNA having the one or more base sequences, a vector containing the recombinant DNA, such as a plasmid and a phage, a transformant transformed by the vector having the DNA, a genetically engineered organism having the DNA introduced therein, and a cell-free protein synthesis system using the DNA as a template for transcription.

The immunoglobulin-binding peptide usable in the affinity separation matrix of the present invention may be obtained as a fusion peptide fused with a publicly-known protein which beneficially has an action to assist the expression of a protein or to facilitate the purification of a protein. In other words, it is possible to obtain a microorganism or cell containing at least one recombinant DNA encoding such a fusion peptide. The example of the above-described protein includes a maltose-binding protein (MBP) and a glutathione S-transferase (GST), but is not restricted thereto.

Site-specific mutagenesis for modifying the DNA encoding the peptide can be carried out by using recombinant DNA technology, PCR method or the like as follows. Specifically, mutagenesis by recombinant DNA technology can be carried out as follows: for example, in the case where there are suitable restriction enzyme recognition sequences on both sides of a target mutagenesis site into which a desired mutation should be inserted in the gene encoding the present invention peptide, cassette mutagenesis method can be carried out in which method a region containing a desired site for mutagenesis is removed by cleaving the restriction enzyme recognition sites with the restriction enzymes and then a mutated DNA fragment is inserted. In the mutated DNA fragment, mutation is introduced only at the target site by chemical synthesis or the like.

For example, site-directed mutagenesis by PCR can be carried out by double primer mutagenesis method. In double primer mutagenesis method, PCR is carried out by using a double-stranded plasmid encoding the peptide as a template, and using two kinds of synthesized oligo primers which contain complementary mutations in the + strand and - strand. A DNA encoding a multimer peptide can be produced by ligating the desired number of DNAs each encoding the monomer peptide (single domain) to one another in tandem. For example, with respect to a connecting method for the DNA encoding a multimer peptide, a suitable restriction enzyme site is introduced in the DNA sequence and double-stranded DNA fragments cleaved with the restriction enzyme are ligated by using a DNA ligase. One restriction enzyme site may be introduced or a plurality of restriction enzyme sites of different types may be introduced. When the base sequences encoding each monomer peptide in the DNA encoding the multimer peptide are the same, homologous recombination may be possibly induced in a host. Thus, the sequence identity between base sequences of DNAs encoding the ligated monomer peptides may be 90% or less, preferably 85% or less, more preferably 80% or less, and even more preferably 75% or less. The identity of the base sequence can be also determined by an ordinary method similarly to the amino acid sequence.

The vector contains a base sequence encoding the above-described peptide or a part of the amino acid sequence of the peptide and a promoter which can be operably linked to the base sequence to function in a host. In general, the expression vector can be obtained by linking or inserting the gene encoding the peptide to a suitable vector. The vector to insert the gene is not particularly restricted as long as the vector is capable of autonomous replication in a host. A plasmid DNA or a phage DNA can be used as such a vector. For example, in the case where Escherichia coli is used as a host, a pQE series vector manufactured by QIAGEN, a pET series vector manufactured by Merck, a pGEX series vector manufactured by GE Healthcare Bioscience or the like can be used.

The transformant can be produced by introducing the recombinant vector of the present invention into a host cell. A method for introducing the recombinant DNA into a host is exemplified by a method using a calcium ion, electroporation method, spheroplast method, lithium acetate method, agrobacterium infection method, particle gun method and polyethylene glycol method, but is not restricted thereto. A method for expressing the function of the obtained gene in a host is also exemplified by a method in which the gene obtained by the present invention is implanted into a genome (chromosome). A host cell is not particularly restricted, and bacteria (eubacteria) such as Escherichia coli, Bacillus subtilis, Brevibacillus, Staphylococcus, Streptococcus, Streptomyces and Corynebacterium can be preferably used in terms of mass production in a low cost.

The immunoglobulin-binding peptide can be produced by cultivating the above-described transformant in a medium to allow the cell to produce and accumulate the present invention peptide in the cultivated cell (including the periplasmic space of the cell) or in the culture liquid outside the cell, and collecting the desired peptide from the culture product. In addition, the peptide can also be produced by cultivating the above-described transformant in a medium to allow the cell to produce and accumulate a fusion protein containing the peptide in the cultivated cell (including the periplasmic space of the cell) or in the culture liquid outside the cell, collecting the fusion peptide from the culture product, cleaving the fusion peptide with a suitable protease, and collecting the desired peptide.

The transformant can be cultivated in a medium in accordance with a common method for cultivating a host cell. The medium used for cultivating the obtained transformant is not particularly restricted as long as the medium enables high yield production of the peptide with high efficiency. Specifically, carbon source and nitrogen source, such as glucose, sucrose, glycerol, polypeptone, meat extract, yeast extract and casamino acid, can be used. In addition, an inorganic salt such as potassium salt, sodium salt, phosphate salt, magnesium salt, manganese salt, zinc salt and iron salt is added as required. In the case of an auxotrophic host cell, a nutritional substance necessary for the growth thereof may be added. In addition, an antibiotic such as penicillin, erythromycin, chloramphenicol and neomycin may be added as required.

Furthermore, in order to inhibit the degradation of the target peptide caused by a host-derived protease present inside or outside the transformant, a publicly-known protease inhibitor and/or other commercially available protease inhibitor may be added in an appropriate concentration. The publicly-known protease inhibitor is exemplified by phenylmethane sulfonyl fluoride (PMSF), benzamidine, 4-(2-aminoethyl)-benzenesulfonyl fluoride (AEBSF), antipain, chymostatin, leupeptin, Pepstatin A, phosphoramidon, aprotinin and ethylenediaminetetraacetic acid (EDTA) .

In order to obtain the rightly folded immunoglobulin-binding peptide, for example, a molecular chaperone such as GroEL/ES, Hsp70/DnaK, Hsp90 and Hsp104/ClpB may be used. For example, such a molecular chaperone is co-existed with the present invention peptide by coexpression or in a form of a fusion protein. As a method for obtaining the rightly folded peptide, addition of an additive for assisting right folding into the medium and cultivation at a low temperature are also exemplified, but the method is not restricted thereto.

The medium for cultivating the transformant produced from an Escherichia coli as a host is exemplified by LB medium containing triptone 1%, yeast extract 0.5% and NaCl 1%, 2xYT medium containing triptone 1.6%, yeast extract 1.0% and NaCl 0.5%, and the like. For example, the transformant may be aerobically cultivated in an aeration-stirring condition at a temperature of 15 to 42°C, preferably 20 to 37°C, for several hours to several days. As a result, the present invention peptide to be obtained is accumulated in the cultivated cell (including the periplasmic space of the cell) or in the culture liquid outside the cell. In some cases, the cultivation may be performed anaerobically without aeration. In the case where a recombinant peptide is secreted, the produced recombinant peptide can be obtained after the cultivation by separating the supernatant containing the secreted peptide by using a common separation method such as centrifugation and filtration from the cultivated cell. In addition, in the case where the peptide is accumulated in the cultivated cell (including the periplasmic space), the peptide accumulated in the cell can be obtained by, for example, collecting the cell from the culture liquid by centrifugation, filtration or the like, and then disrupting the cell by sonication, French press method or the like, and/or solubilizing the cell by adding a surfactant or the like.

A method for purifying the peptide can be carried out by any one or an appropriate combination of techniques such as affinity chromatography, cation or anion exchange chromatography and gel filtration chromatography. It can be confirmed whether the obtained purified substance is the target peptide or not by an ordinary method such as SDS polyacrylamide gel electrophoresis, N-terminal amino acid sequence analysis and Western blot analysis.

The present application claims the benefit of the priority date of Japanese patent application No. 2017-71909 filed on March 31, 2017. All of the contents of the Japanese patent application No. 2017-71909 filed on March 31, 2017, are incorporated by reference herein.

### EXAMPLES

Hereinafter, the present invention is described in more detail with Examples. The present invention is however not restricted to the following Examples. Three amino acid sequences of immunoglobulin-binding domains of publically-known wild SpG (SEQ ID NOs: 17 to 19) and one amino acid sequence of an SpG mutant of which binding ability to a Fab region was increased (SEQ ID NO: 20) were used as amino acid sequences to be compared.

### Example 1: Preparation of immunoglobulin-binding peptides

### (1) Preparation of expression plasmids of immunoglobulin-binding peptides

The DNA sequences encoding the immunoglobulin-binding peptides were designed by reverse translation from the amino acid sequences of the peptides. PCR was carried out by using three kinds of single strand oligo DNA (f31/f32/f33), and the PCR product was digested by using restriction enzymes BamHI/EcoRI to be integrated in the multi cloning site of an expression vector pGEX-6P-1 treated by the same restriction enzymes. In the PCR to prepare the encoding DNA, a small amount of f32 (0.2 µM, leading) and f33 (10 µM, lagging) were firstly extended by overlap extension PCR, and then double-stranded DNA formed by overlap extension PCR using f31 (10 µM, leading) and f33 (lagging) extended by the former reaction became an encoding DNA sequence having restriction enzyme sites at both ends. Various oligo DNA sequences corresponding to f31 to f33 of each PG derivatives were designed to proceed the reaction, and the synthesis thereof was outsourced to Eurofins. With respect to the specific experimental procedure, PCR was carried out by using BlendTaq Plus (TOYOBO) as a polymerase. The PCR product was subjected to agarose electrophoresis and the target band was cut out to extract double-stranded DNA. The double-stranded DNA was cleaved with the restriction enzymes BamHI and Eco52I (Takara Bio). Then, a plasmid vector pGEX-6P-1 (GE Healthcare Bioscience) was similarly treated by BamHI/EcoRI, and then subjected to dephosphorylation treatment by a dephosphorylation enzyme CIAP (Takara Bio) and ligation reaction using Ligation high (TOYOBO).

The combinations of the coding DNA sequence corresponding to the amino acid sequence of the immunoglobulin-binding peptides and the oligo DNA sequence used for preparing the expression vector (f31 to f33) are shown in Table 1.

**Table 1**

| Amino acid sequence | Encoding DNA | Oligo DNA f31 | Oligo DNA f32 | Oligo DNA f33 |
|---|---|---|---|---|
| 2 | 21 | 36 | 37 | 38 |
| 3 | 22 | 39 | 40 | 38 |
| 4 | 23 | 39 | 41 | 42 |
| 5 | 24 | 39 | 43 | 44 |
| 6 | 25 | 45 | 46 | 47 |
| 7 | 26 | 48 | 49 | 50 |
| 8 | 27 | 51 | 52 | 38 |
| 9 | 28 | 53 | 54 | 38 |
| 10 | 29 | 53 | 55 | 42 |
| 11 | 30 | 53 | 56 | 44 |
| 12 | 31 | 57 | 58 | 47 |
| 13 | 32 | 59 | 60 | 50 |
| 14 | 33 | 61 | 54 | 38 |
| 15 | 34 | 61 | 55 | 42 |
| 16 | 35 | 61 | 56 | 44 |

A competent cell ("Escherichia coli HB101" manufactured by Takara Bio, Inc.) was transformed by using the above-described plasmid vector pGEX-6P-1 in accordance with the protocol attached to the competent cell product. When the plasmid vector pGEX-6P-1 is used, a peptide which is fused with glutathione-S-transferase (hereinafter, abbreviated as "GST") can be produced. Then, the plasmid DNA was amplified and extracted by using a plasmid purification kit ("Wizard Plus SV Minipreps DNA Purification System" manufactured by Promega) in accordance with the standard protocol attached to the kit. The base sequence of the encoding DNA of the expression plasmid was determined by using a DNA sequencer ("3130xl Genetic Analyzer" manufactured by Applied Biosystems). The sequencing PCR was performed by using a gene analysis kit ("BigDye Terminator v. 1.1 Cycle Sequencing Kit" manufactured by Applied Biosystems) and DNA primers for sequencing the plasmid vector pGEX-6P-1 (GE Healthcare Bioscience) in accordance with the attached protocol. The sequencing product was purified by using a plasmid purification kit ("BigDye XTerminator Purification Kit" manufactured by Applied Biosystems) in accordance with the attached protocol and used for the base sequence analysis.

### (2) Preparation of immunoglobulin-binding peptides

The transformant cell produced by integrating each of the immunoglobulin-binding peptides obtained in the above-described (1) was cultivated in 2xYT medium containing ampicillin at 37°C overnight. The culture liquid was inoculated in 2xYT medium containing about a 100-fold amount of ampicillin and cultivated at 37°C for about 1 hour and then at 25°C for about 1 hour. Then, isopropyl-1-thio-β-D-galactoside (IPTG) was added so that the final concentration thereof became 0.1 mM, and the transformant was further cultivated at 25°C for about 18 hours.

After the cultivation, the cell was collected by centrifugation and re-suspended in 5 mL of PBS buffer. The cell was broken by sonication and centrifuged to separate a supernatant fraction as a cell-free extract and an insoluble fraction. When a target gene is integrated into the multiple cloning site of pGEX-6P-1 vector, a fusion peptide having GST added to the N-terminal is produced. Each fraction was analyzed by SDS electrophoresis; as a result, a peptide band assumed to be induced by IPTG was detected at a position corresponding to a molecular weight of about 25,000 or more in all of the cell-free extracts obtained from each of culture liquid of each transformant. The molecular weights were approximately similar but the positions of bands were different depending on the kind of immunoglobulin-binding peptides.

The GST fusion peptide was roughly purified from each of the cell-free extract containing the GST fusion peptide by affinity chromatography using a GSTrap FF column (GE Healthcare Bioscience), which has an affinity for GST. Each of the cell-free extract was added to the GSTrap FF column and the column was washed with a standard buffer (20 mM NaH₂PO₄-Na₂HPO₄, 150 mM NaCl, pH 7.4). Then, the target GST fusion peptide was eluted by using an elution buffer (50 mM Tris-HCl, 20 mM glutathione, pH 8.0). As the sample used for assay in the following Examples with fusing GST, a peptide solution obtained by concentrating the eluent and replacing the elution buffer with the standard buffer by using centrifugal filter unit Amicon (Merck Millipore) was used.

When a gene is integrated into a multiple cloning site of pGEX-6P-1 vector, an amino acid sequence by which GST can be removed by using sequence-specific protease, "PreScission Protease" (GE Healthcare Bioscience), is inserted between GST and a target peptide. By using such PreScission Protease, GST was removed in accordance with the attached protocol. The target peptide was purified by gel filtration chromatography using Superdex 75 10/300 GL column (GE Healthcare Bioscience) from the GST-removed sample. Each of the reaction mixture was added to the Superdex 75 10/300 GL column equilibrated with the standard buffer, and the target peptide was separated and purified from the removed GST and PreScission Protease. The above-described all of the peptide purification by chromatography using the column was performed by using AKTAprime plus system (GE Healthcare Bioscience). In addition, after the removal of GST, the peptide produced in the present Example had the sequence of Gly-Pro-Leu-Gly-Ser derived from the vector pGEX-6P-1 at the N-terminal side.

### Example 2: Evaluation of binding ability of immunoglobulin-binding peptides

### (1) Preparation of immunoglobulins

An IgG product derived from human blood was used as a raw material to prepare an IgG solution, and the IgG was fragmented into a Fab fragment and an Fc fragment by using papain. Hereinafter, a Fab fragment is simply abbreviated as "Fab" and an Fc fragment is simply abbreviated as "Fc". The Fab and Fc were separated to be purified to prepare a Fab solution and an Fc solution. Specifically, a blood plasma fraction product γ-GLOBULIN for I.M. injection 1500 mg/10mL "NICHIYAKU" was dissolved in a buffer for digestion by papain (0.1 M AcOH-AcONa, 2 mM EDTA, 1 mM cysteine, pH 5.5), and agarose on which papain was immobilized ("Papain Agarose from papaya latex" manufactured by SIGMA) was added thereto. The mixture was incubated with stirring by a rotator at 37°C for about 8 hours. The Fab was purified from the Fc by recovering the Fab as a flow-through fraction in an affinity chromatography using KanCapA column (KANEKA Corporation) from the reaction mixture which contained both of the Fab and Fc and which was separated from the agarose on which papain was immobilized. The Fc adsorbed on the column was eluted by 0.05 M acetic acid/sodium acetate buffer (pH 3.5) and immediately neutralized by 0.1 M Tris-HCl buffer (pH 8.0). In addition, a solution containing IgG was prepared similarly to Fc by subjecting a blood plasma fraction product γ-GLOBULIN to a similar chromatography procedure using KanCapA without a Papain digestion treatment. Each solution of IgG, Fab and Fc was subjected to purification by gel filtration chromatography using Superdex 75 10/300 GL column (GE Healthcare) to obtain solutions of immunoglobulin derivatives. In the chromatography, the standard buffer was used for equilibration and separation. Similarly to the above-described Example 1, AKTAprime plus system was used in the chromatography for protein purification.

### (2) Measurement of binding response of peptides to immunoglobulin derivatives

A binding ability of immunoglobulin-binding peptides to immunoglobulin derivatives was measured by an evaluation system using Biosensor Octet (Pall Corporation) and SA biosensor. SA biosensor is recommended as the most standard evaluation system in Octet, and a ligand is immobilized in SA biosensor by using streptavidin-biotin binding.

The concentration of IgG obtained in the above (1) was adjusted to 3 mg/mL, and the concentrations of Fab and Fc were adjusted to 1 mg/mL. To 1 mL of each solution, 20 µL of 1 mM aqueous solution of amine-reactive biotinylation reagent "EZ-link-NHS-PEG4-Biotin" (Themo Fisher) was added. The mixture was incubated overnight to prepare a biotinylated IgG, a biotinylated Fab and a biotinylated Fc. The biotinylated IgG, biotinylated Fab and biotinylated Fc was diluted so that the final concentration became about 0.1 mg/mL by using a running buffer (20 mM NaH₂PO₄-Na₂HPO₄, 150 mM NaCl, pH 7.4) and similarly contacted with eight SA biosensors for 600 seconds. All of the rotational speeds during the contact were set to 1000 rpm. Then, a cycle of a contact with 50 mM citrate, pH 2.4 for 10 second and a contact with the running buffer for 10 seconds was repeated 3 times as a washing-regeneration step. It was confirmed that streptavidin-biotin binding was not dissociated in this condition. The evaluation system was designed so that the same SA biosensor was used with respect to one immunoglobulin. The data of an association phase to contact with each peptide solution for 120 seconds and then a dissociation phase to contact with the standard buffer for 120 seconds were obtained. The measurement was repeatedly carried out with respect to the three kinds of data of the cases where the concentration of the peptide was 10 nM, 100 nM and 1000 nM through the intermediary of the above-described washing-regeneration step by using the same SA biosensor for the same kind of the peptide. The above-described measurement is shown in Table 2.

**Table 2**

| | Step | Solution | Assay time | Cycle |
|---|---|---|---|---|
| 1 | Baseline | Standard buffer | 120sec | Only initially |
| 2 | Loading | Biotinylated IgG/Fab/Fc solution 0.1 mg/mL | 600sec | |
| 3 | Regeneration | 50mM Citrate-HCl, pH2.4 → Standard buffer | (10s→10s) × 3 total 60sec | |
| 4 | Baseline | Standard buffer | 300sec | Repeated for each peptide solution |
| 5 | Association | Each peptide solution (10nM/100nM/1000nM) | 120sec | |
| 6 | Dissociation | Standard buffer | 120sec | |
| 7 | Regeneration | Same as Step 3 | Same as Step 3 | |

A fitting analysis was carried out with a standard binding model of 1 : 1 using an attached analysis software. An association constant (K_{A} = k_{on/}k_{off}), an association rate constant (kₒₙ) and a dissociation rate constant (k_{off}) were calculated, and common logarithm Log 10 thereof are demonstrated in graphs. When the value is one digit different, the parameter means 1 order (10 times) different. Since a binding becomes strong in the case of a smaller dissociation constant, the axis of the graph is inverted. Thus, it is demonstrated in the graphs that the bar is larger on the right, a binding means strong with respect to each parameter.

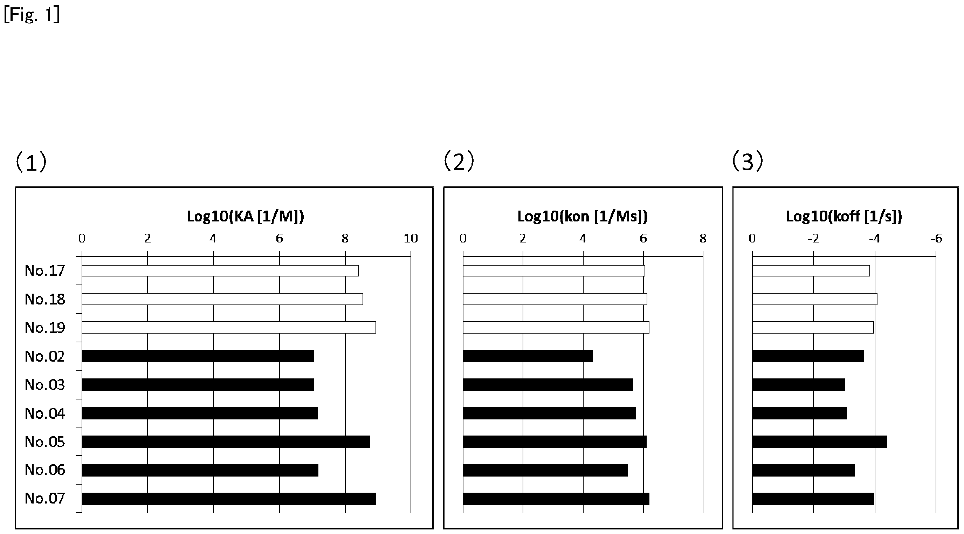

The graphs to demonstrate the IgG-binding parameters of immunoglobulin-binding peptides of SEQ ID NOs: 2 to 7 for the comparison with the peptides of IgG-binding domains of SpG of SEQ ID NOs: 17 to 19 are shown as Figure 1. It was confirmed that all of the immunoglobulin-binding peptides according to the present invention have an IgG-binding ability similarly to the IgG-binding domain of SpG. An affinity separation matrix having an improved IgG-grasping performance may be developed by using a peptide exhibiting superior value to SpG as a ligand. On the one hand, an affinity separation matrix from which IgG can be easily recovered may be developed by using a peptide exhibiting smaller value than SpG as a ligand. Thus, it is considered to be important that the present invention peptide exhibits an association constant of the same order as SpG.

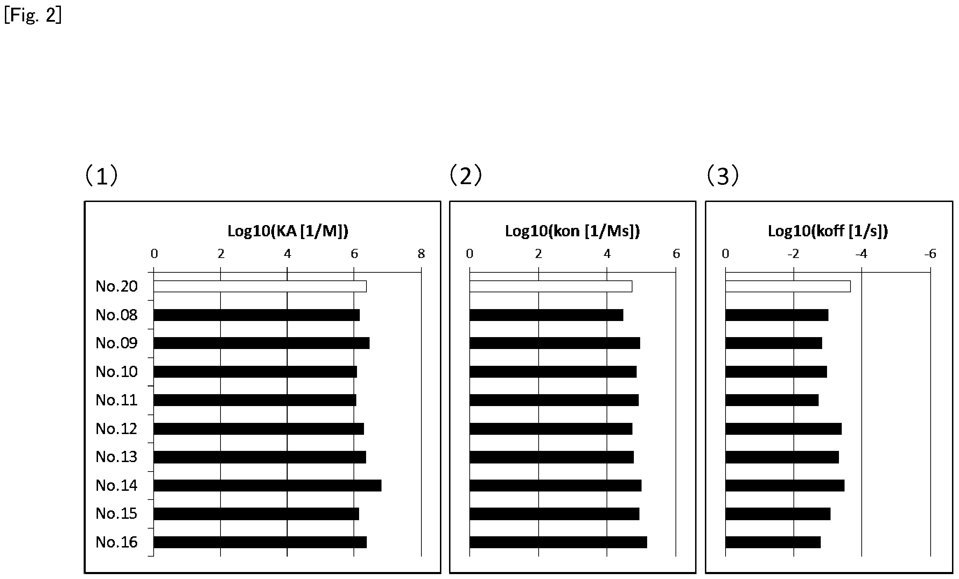

The graphs to demonstrate the Fab-binding parameters of immunoglobulin-binding peptides of SEQ ID NOs: 8 to 16 for the comparison with the Fab-binding-enhanced mutant having SEQ ID NO: 20 of the IgG-binding domain of SpG as Comparative example 1 are shown as Figure 2. It was confirmed as the above that all of the immunoglobulin-binding peptides according to the present invention have a sufficient IgG-binding ability as a binding peptide to a Fab of an immunoglobulin. The peptides of SEQ ID NOs: 8 to 16 had amino acid sequences of partly mutated SEQ ID NOs: 2 to 7 with reference to Patent documents 2 to 4. It can be said by the data that various amino acid sequences of SEQ ID NO: 1 is basically included in the present invention range.

### Example 3: Evaluation of alkali resistance of immunoglobulin-binding peptide

### (1) Alkali treatment of peptides

Each peptide was subjected to dialysis using ultrapure water and the concentration thereof was adjusted to obtain 4 µM aqueous solution. To 0.2 mL of the aqueous solution, 0.1 mL corresponding to a half amount of the aqueous solution of 45 mM sodium hydroxide aqueous was added. The mixture was incubated at 25°C for 2 hours and then neutralized by 0.1 mL of 50 mM acetic acid (pH 3.0). After it was confirmed that the mixture was neutralized by using a pH test paper, the mixture was diluted to 20 times by the standard buffer so that the concentration was adjusted to 100 nM.

The peptide solution prepared as the above was defined as a sample before alkali treatment at 0 hr, and 0.1 mL of the above sodium hydroxide aqueous solution and 0.1 mL of 50 mM acetic acid (pH 3.0) were preliminarily mixed and the mixture was added to the sample. The final concentration of the peptide in the solution was 100 nM.

### (2) Residual immunoglobulin-binding activity of peptides after alkali treatment

A binding to an immunoglobulin by a similar method to Example 2(2) using Octet and the peptide solutions before and after the alkali treatment prepared in the above (1) was measured. An Fc was used instead of IgG in this experiment in order to simplify a binding pattern. It could be assumed that a change of a binding signal value due to concentration difference was broadly similar, since it could be confirmed that binding constants were included in the same order. Thus, a ratio of a binding signal value in the condition of N = 3 after the alkali treatment to a binding signal value before the alkali treatment was calculated as a residual binding activity to be demonstrated in a graph.

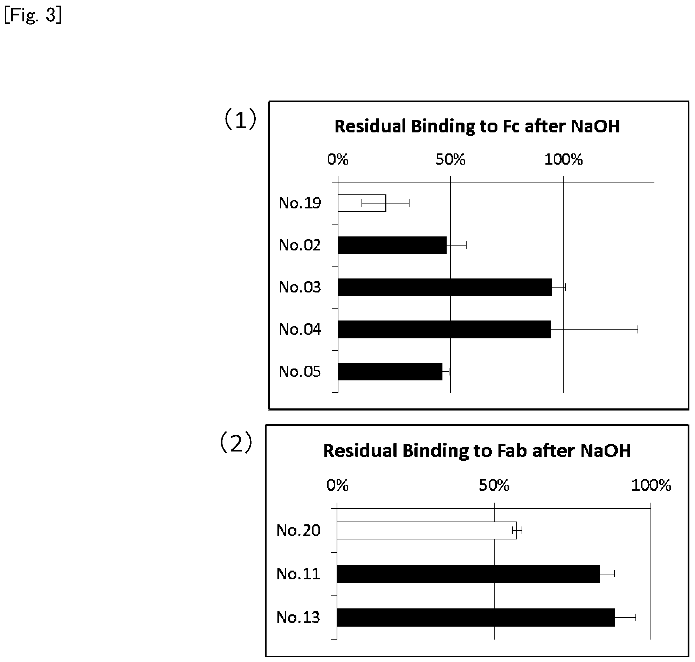

The graph to demonstrate the residual binding activity to an Fc of immunoglobulin-binding peptides of SEQ ID NOs: 2 to 5 after the alkali treatment for the comparison with the IgG-binding domain peptide of SpG of SEQ ID NO: 19 as Comparative example 1 is shown as Figure 3(1). It was confirmed as shown in the graph that the present invention peptides have significantly higher alkali resistance than the IgG-binding domain of SpG.

A residual binding activity to a Fab of the Fab-binding activity-enhanced peptides of SEQ ID NOs: 11 and 13 after the alkali resistance was also evaluated. The result in comparison with the Fab-binding activity-enhanced mutant (SEQ ID NO: 20) of the IgG-binding domain of SpG as Comparative example 1 is shown as Figure 3(2). It was confirmed that the alkali resistance of the Fab-binding activity-enhanced peptide obtained in the present invention is similarly improved.

There was variability among numerical values but it is natural that such a degree of variation occurs, since a nonspecific damage due to an alkali was evaluated and this evaluation system was sensitive to the damage. In addition, there were clear significant differences between average values. The result is, therefore, considered to have high reliability and it can be said that the present invention keeps higher level than comparative controls. The affinity separation matrix having the peptide evaluated in the present Example as a ligand must have a resistant property to damage by an alkaline washing.

### Comparative example 1

With respect to the proteins of SEQ ID NOs: 17 to 20 used as comparative controls in the present Example, an expression plasmid was prepared by a similar method to Example 1(1) using oligo DNAs of SEQ ID NOs shown in Table 3. Then, a protein sample was prepared by a similar method to Example 1(2) and evaluated with the peptides of the present invention by a similar method to Example 2 and Example 3.

**Table 3**

| Amino acid sequence | Encoding DNA | Oligo DNA f31 | Oligo DNA f32 | Oligo DNA f33 |
|---|---|---|---|---|
| 17 | 62 | 66 | 67 | 68 |
| 18 | 63 | 69 | 67 | 68 |
| 19 | 64 | 70 | 71 | 72 |
| 20 | 65 | 73 | 74 | 72 |

## Claims

1. An affinity separation matrix,
comprising an immunoglobulin-binding peptide and a water-insoluble carrier,
wherein the immunoglobulin-binding peptide comprises an amino acid sequence of SEQ ID NO: 1 or an amino acid sequence with a sequence identity of 94% or more to the amino acid sequence of SEQ ID NO: 1, and
the immunoglobulin-binding peptide is immobilized on the water-insoluble carrier as a ligand.

2. The affinity separation matrix according to claim 1, wherein the amino acid sequence of SEQ ID NO: 1 is any one of amino acid sequences of SEQ ID NOs: 2 to 16.

3. The affinity separation matrix according to claim 1, wherein the immunoglobulin-binding peptide comprises an amino acid sequence of SEQ ID NO: 1 with deletion, substitution and/or addition of one or more amino acids at N-terminal and/or C-terminal.

4. The affinity separation matrix according to any one of claims 1 to 3, wherein an immunoglobulin-binding peptide multimer comprising two or more of the immunoglobulin-binding peptides connected to one another is immobilized on the water-insoluble carrier as the ligand.

5. A method for producing a protein comprising an Fc region and/or a Fab region of an immunoglobulin, comprising:
contacting a liquid sample comprising the protein with the affinity separation matrix according to any one of claims 1 to 4, and
separating the protein adsorbed on the affinity separation matrix from the affinity separation matrix.


SEQUENCE LISTING

<110>  Kaneka Corporation

<120>  Affinity separation matrix of immunoglobulin

<130>  B170200

<150>  JP 2017-071909
<151>  2017-03-31

<160>  74

<170>  PatentIn version 3.5

<210>  1
<211>  56
<212>  PRT
<213>  Artificial Sequence (SpG-like Novel Peptide)

<220>
<223>  SpG-like Novel Peptide


<220>
<221>  MISC_FEATURE
<222>  (1)..(1)
<223>  Xaa at position 1 is Asp or Thr

<220>
<221>  MISC_FEATURE
<222>  (2)..(2)
<223>  Xaa at position 2 is Thr or Ser

<220>
<221>  MISC_FEATURE
<222>  (6)..(6)
<223>  Xaa at position 6 is Val or Ile

<220>
<221>  MISC_FEATURE
<222>  (10)..(10)
<223>  Xaa at position 10 is Ala, Val or Asn

<220>
<221>  MISC_FEATURE
<222>  (12)..(12)
<223>  Xaa at position 12 is Phe or Leu

<220>
<221>  MISC_FEATURE
<222>  (13)..(13)
<223>  Xaa at position 13 is Ser or Thr

<220>
<221>  MISC_FEATURE
<222>  (15)..(15)
<223>  Xaa at position 13 is Glu or Tyr

<220>
<221>  MISC_FEATURE
<222>  (17)..(17)
<223>  Xaa at position 17 is Thr or Ala

<220>
<221>  MISC_FEATURE
<222>  (19)..(19)
<223>  Xaa at position 19 is Lys or Ile

<220>
<221>  MISC_FEATURE
<222>  (21)..(21)
<223>  Xaa at position 21 is Val or Ala

<220>
<221>  MISC_FEATURE
<222>  (23)..(23)
<223>  Xaa at position 23 is Ala or Thr

<220>
<221>  MISC_FEATURE
<222>  (24)..(24)
<223>  Xaa at position 24 is Ala or Glu

<220>
<221>  MISC_FEATURE
<222>  (25)..(25)
<223>  Xaa at position 25 is Thr or Val

<220>
<221>  MISC_FEATURE
<222>  (28)..(28)
<223>  Xaa at position 28 is Lys or Gln

<220>
<221>  MISC_FEATURE
<222>  (29)..(29)
<223>  Xaa at position 29 is Ala, Glu or Thr

<220>
<221>  MISC_FEATURE
<222>  (30)..(30)
<223>  Xaa at position 30 is Phe or Leu

<220>
<221>  MISC_FEATURE
<222>  (31)..(31)
<223>  Xaa at position 31 is Lys or Arg

<220>
<221>  MISC_FEATURE
<222>  (32)..(32)
<223>  Xaa at position 32 is Gln or Asp

<220>
<221>  MISC_FEATURE
<222>  (33)..(33)
<223>  Xaa at position 33 is Tyr or Phe

<220>
<221>  MISC_FEATURE
<222>  (35)..(35)
<223>  Xaa at position 35 is Thr, Asn or Phe

<220>
<221>  MISC_FEATURE

<222>  (36)..(36)
<223>  Xaa at position 36 is Ala, Asp, Glu or Lys

<220>
<221>  MISC_FEATURE
<222>  (38)..(38)
<223>  Xaa at position 38 is Asn or Gly

<220>
<221>  MISC_FEATURE
<222>  (39)..(39)
<223>  Xaa at position 39 is Val or Thr

<220>
<221>  MISC_FEATURE
<222>  (40)..(40)
<223>  Xaa at position 40 is Asp, Tyr or Thr

<220>
<221>  MISC_FEATURE
<222>  (42)..(42)
<223>  Xaa at position 42 is Glu or Val

<220>
<221>  MISC_FEATURE
<222>  (44)..(44)
<223>  Xaa at position 44 is Ser or Ala

<220>
<221>  MISC_FEATURE
<222>  (47)..(47)
<223>  Xaa at position 47 is Asp, Ala or Thr

<400>  1

Xaa Xaa Tyr Lys Leu Xaa Val Lys Gly Xaa Thr Xaa Xaa Gly Xaa Thr
1               5                   10                  15

Xaa Thr Xaa Ala Xaa Asp Xaa Xaa Xaa Ala Glu Xaa Xaa Xaa Xaa Xaa
            20                  25                  30

Xaa Ala Xaa Xaa Asn Xaa Xaa Xaa Gly Xaa Trp Xaa Tyr Asp Xaa Ala
        35                  40                  45

Thr Lys Thr Phe Thr Val Thr Glu
    50                  55

<210>  2
<211>  56
<212>  PRT
<213>  Streptococcus dysgalactiae

<400>  2

Thr Thr Tyr Lys Leu Ile Val Lys Gly Asn Thr Phe Ser Gly Glu Thr
1               5                   10                  15

Thr Thr Lys Ala Val Asp Ala Glu Thr Ala Glu Lys Ala Phe Lys Gln

20                  25                  30

Tyr Ala Thr Ala Asn Asn Val Asp Gly Glu Trp Ser Tyr Asp Asp Ala
        35                  40                  45

Thr Lys Thr Phe Thr Val Thr Glu
    50                  55

<210>  3
<211>  56
<212>  PRT
<213>  Streptococcus dysgalactiae

<400>  3

Thr Thr Tyr Lys Leu Ile Val Lys Gly Asn Thr Phe Ser Gly Glu Thr
1               5                   10                  15

Thr Thr Lys Ala Val Asp Ala Glu Thr Ala Glu Lys Ala Phe Lys Gln
            20                  25                  30

Tyr Ala Thr Ala Asn Asn Val Asp Gly Glu Trp Ser Tyr Asp Asp Ala
        35                  40                  45

Thr Lys Thr Phe Thr Val Thr Glu
    50                  55

<210>  4
<211>  56
<212>  PRT
<213>  Streptococcus dysgalactiae

<400>  4

Thr Thr Tyr Lys Leu Ile Val Lys Gly Asn Thr Phe Ser Gly Glu Thr
1               5                   10                  15

Thr Thr Lys Ala Ile Asp Ala Ala Thr Ala Glu Lys Glu Phe Lys Gln
            20                  25                  30

Tyr Ala Thr Ala Asn Gly Val Asp Gly Glu Trp Ser Tyr Asp Asp Ala
        35                  40                  45

Thr Lys Thr Phe Thr Val Thr Glu
    50                  55

<210>  5
<211>  56
<212>  PRT
<213>  Streptococcus dysgalactiae

<400>  5

Thr Thr Tyr Lys Leu Ile Val Lys Gly Asn Thr Phe Ser Gly Glu Thr
1               5                   10                  15

Thr Thr Lys Ala Val Asp Ala Glu Thr Ala Glu Lys Ala Phe Lys Gln
            20                  25                  30

Tyr Ala Asn Glu Asn Gly Val Tyr Gly Glu Trp Ser Tyr Asp Asp Ala
        35                  40                  45

Thr Lys Thr Phe Thr Val Thr Glu
    50                  55

<210>  6
<211>  56
<212>  PRT
<213>  Streptococcus zooepidemicus

<400>  6

Thr Ser Tyr Lys Leu Val Ile Lys Gly Ala Thr Phe Ser Gly Glu Thr
1               5                   10                  15

Ala Thr Lys Ala Val Asp Ala Ala Val Ala Glu Gln Thr Phe Arg Asp
            20                  25                  30

Tyr Ala Asn Lys Asn Gly Val Asp Gly Val Trp Ala Tyr Asp Ala Ala
        35                  40                  45

Thr Lys Thr Phe Thr Val Thr Glu
    50                  55

<210>  7
<211>  56
<212>  PRT
<213>  Streptococcus zooepidemicus

<400>  7

Thr Thr Tyr Arg Leu Val Ile Lys Gly Val Thr Phe Ser Gly Glu Thr
1               5                   10                  15

Ala Thr Lys Ala Val Asp Ala Ala Thr Ala Glu Gln Thr Phe Arg Gln
            20                  25                  30

Tyr Ala Asn Asp Asn Gly Ile Thr Gly Glu Trp Ala Tyr Asp Thr Ala
        35                  40                  45

Thr Lys Thr Phe Thr Val Thr Glu
    50                  55

<210>  8
<211>  56
<212>  PRT
<213>  Artificial Sequence

<220>
<223>  novel peptide

<400>  8

Thr Thr Tyr Lys Leu Val Val Lys Gly Asn Thr Leu Thr Gly Tyr Thr
1               5                   10                  15

Thr Thr Ile Ala Ile Asp Thr Ala Thr Ala Glu Lys Glu Leu Lys Gln
            20                  25                  30

Phe Ala Thr Ala Asn Asn Val Asp Gly Glu Trp Ser Tyr Asp Asp Ala
        35                  40                  45

Thr Lys Thr Phe Thr Val Thr Glu
    50                  55

<210>  9
<211>  56
<212>  PRT
<213>  Artificial Sequence

<220>
<223>  novel peptide

<400>  9

Thr Thr Tyr Lys Leu Ile Val Lys Gly Asn Thr Leu Thr Gly Tyr Thr
1               5                   10                  15

Thr Thr Ile Ala Val Asp Ala Glu Thr Ala Glu Lys Ala Leu Lys Gln
            20                  25                  30

Phe Ala Thr Ala Asn Asn Val Asp Gly Glu Trp Ser Tyr Asp Asp Ala
        35                  40                  45

Thr Lys Thr Phe Thr Val Thr Glu
    50                  55

<210>  10
<211>  56
<212>  PRT
<213>  Artificial Sequence

<220>
<223>  novel peptide

<400>  10

Thr Thr Tyr Lys Leu Ile Val Lys Gly Asn Thr Leu Thr Gly Tyr Thr

1               5                   10                  15

Thr Thr Ile Ala Ile Asp Ala Ala Thr Ala Glu Lys Glu Leu Lys Gln
            20                  25                  30

Phe Ala Thr Ala Asn Gly Val Asp Gly Glu Trp Ser Tyr Asp Asp Ala
        35                  40                  45

Thr Lys Thr Phe Thr Val Thr Glu
    50                  55

<210>  11
<211>  56
<212>  PRT
<213>  Artificial Sequence

<220>
<223>  novel peptide

<400>  11

Thr Thr Tyr Lys Leu Ile Val Lys Gly Asn Thr Leu Thr Gly Tyr Thr
1               5                   10                  15

Thr Thr Ile Ala Val Asp Ala Glu Thr Ala Glu Lys Ala Leu Lys Gln
            20                  25                  30

Phe Ala Asn Glu Asn Gly Val Tyr Gly Glu Trp Ser Tyr Asp Asp Ala
        35                  40                  45

Thr Lys Thr Phe Thr Val Thr Glu
    50                  55

<210>  12
<211>  56
<212>  PRT
<213>  Artificial Sequence

<220>
<223>  novel peptide

<400>  12

Thr Ser Tyr Lys Leu Val Ile Lys Gly Ala Thr Leu Thr Gly Tyr Thr
1               5                   10                  15

Ala Thr Ile Ala Val Asp Ala Ala Val Ala Glu Gln Thr Leu Arg Asp
            20                  25                  30

Phe Ala Asn Lys Asn Gly Val Asp Gly Val Trp Ala Tyr Asp Ala Ala
        35                  40                  45

Thr Lys Thr Phe Thr Val Thr Glu
    50                  55

<210>  13
<211>  56
<212>  PRT
<213>  Artificial Sequence

<220>
<223>  novel peptide

<400>  13

Thr Thr Tyr Arg Leu Val Ile Lys Gly Val Thr Leu Thr Gly Tyr Thr
1               5                   10                  15

Ala Thr Ile Ala Val Asp Ala Ala Thr Ala Glu Gln Thr Leu Arg Gln
            20                  25                  30

Phe Ala Asn Asp Asn Gly Ile Thr Gly Glu Trp Ala Tyr Asp Thr Ala
        35                  40                  45

Thr Lys Thr Phe Thr Val Thr Glu
    50                  55

<210>  14
<211>  56
<212>  PRT
<213>  Artificial Sequence

<220>
<223>  novel peptide

<400>  14

Thr Thr Tyr Lys Leu Ile Val Lys Gly Asn Thr Leu Ser Gly Tyr Thr
1               5                   10                  15

Thr Thr Ile Ala Val Asp Ala Glu Thr Ala Glu Lys Ala Leu Lys Gln
            20                  25                  30

Phe Ala Thr Ala Asn Asn Val Asp Gly Glu Trp Ser Tyr Asp Asp Ala
        35                  40                  45

Thr Lys Thr Phe Thr Val Thr Glu
    50                  55

<210>  15
<211>  56
<212>  PRT
<213>  Artificial Sequence

<220>
<223>  novel peptide

<400>  15

Thr Thr Tyr Lys Leu Ile Val Lys Gly Asn Thr Leu Ser Gly Tyr Thr
1               5                   10                  15

Thr Thr Ile Ala Ile Asp Ala Ala Thr Ala Glu Lys Glu Leu Lys Gln
            20                  25                  30

Phe Ala Thr Ala Asn Gly Val Asp Gly Glu Trp Ser Tyr Asp Asp Ala
        35                  40                  45

Thr Lys Thr Phe Thr Val Thr Glu
    50                  55

<210>  16
<211>  56
<212>  PRT
<213>  Artificial Sequence

<220>
<223>  novel peptide

<400>  16

Thr Thr Tyr Lys Leu Ile Val Lys Gly Asn Thr Leu Ser Gly Tyr Thr
1               5                   10                  15

Thr Thr Ile Ala Val Asp Ala Glu Thr Ala Glu Lys Ala Leu Lys Gln
            20                  25                  30

Phe Ala Asn Glu Asn Gly Val Tyr Gly Glu Trp Ser Tyr Asp Asp Ala
        35                  40                  45

Thr Lys Thr Phe Thr Val Thr Glu
    50                  55

<210>  17
<211>  56
<212>  PRT
<213>  Streptococcus mutans

<400>  17

Thr Thr Tyr Lys Leu Ile Leu Asn Gly Lys Thr Leu Lys Gly Glu Thr
1               5                   10                  15

Thr Thr Glu Ala Val Asp Ala Ala Thr Ala Glu Lys Val Phe Lys Gln
            20                  25                  30

Tyr Ala Asn Asp Asn Gly Val Asp Gly Glu Trp Thr Tyr Asp Asp Ala
        35                  40                  45

Thr Lys Thr Phe Thr Val Thr Glu
    50                  55

<210>  18
<211>  56
<212>  PRT
<213>  Streptococcus mutans

<400>  18

Thr Thr Tyr Lys Leu Val Ile Asn Gly Lys Thr Leu Lys Gly Glu Thr
1               5                   10                  15

Thr Thr Glu Ala Val Asp Ala Ala Thr Ala Glu Lys Val Phe Lys Gln
            20                  25                  30

Tyr Ala Asn Asp Asn Gly Val Asp Gly Glu Trp Thr Tyr Asp Asp Ala
        35                  40                  45

Thr Lys Thr Phe Thr Val Thr Glu
    50                  55

<210>  19
<211>  56
<212>  PRT
<213>  Streptococcus mutans

<400>  19

Thr Thr Tyr Lys Leu Val Ile Asn Gly Lys Thr Leu Lys Gly Glu Thr
1               5                   10                  15

Thr Thr Lys Ala Val Asp Ala Glu Thr Ala Glu Lys Ala Phe Lys Gln
            20                  25                  30

Tyr Ala Asn Asp Asn Gly Val Asp Gly Val Trp Thr Tyr Asp Asp Ala
        35                  40                  45

Thr Lys Thr Phe Thr Val Thr Glu
    50                  55

<210>  20
<211>  56
<212>  PRT
<213>  Artificial Sequence

<220>
<223>  novel peptide

<400>  20

Thr Thr Tyr Lys Leu Val Ile Asn Gly Lys Thr Leu Thr Gly Tyr Thr
1               5                   10                  15

Thr Thr Ile Ala Val Asp Ala Glu Thr Ala Glu Lys Ala Leu Lys Gln
            20                  25                  30

Phe Ala Asn Asp Asn Gly Val Asp Gly Val Trp Thr Tyr Asp Asp Ala
        35                  40                  45

Thr Lys Thr Phe Thr Val Thr Glu
    50                  55

<210>  21
<211>  171
<212>  DNA
<213>  Artificial Sequence

<220>
<223>  code DNA

<400>  21
accacctaca aactggtcgt gaaaggtaat agctttaaag gcgagactac gaccaaagcc        60

attgatactg ctacggccga gaaagagttc aaacagtacg ctaccgctaa taacgtcgac       120

ggtgaatgga gctacgacga cgctaccaaa accttcacgg ttaccgaata a                171

<210>  22
<211>  171
<212>  DNA
<213>  Artificial Sequence

<220>
<223>  code DNA

<400>  22
accacctaca aactgatcgt gaaaggtaat acctttagcg gcgagactac gaccaaagcc        60

gttgatgctg aaacggccga gaaagcgttc aaacagtacg ctaccgctaa taacgtcgac       120

ggtgaatgga gctacgacga cgctaccaaa accttcacgg ttaccgaata a                171

<210>  23
<211>  171
<212>  DNA
<213>  Artificial Sequence

<220>
<223>  code DNA

<400>  23
accacctaca aactgatcgt gaaaggtaat acctttagcg gcgagactac gaccaaagcc        60

attgatgctg ctacggccga gaaagagttc aaacagtacg ctaccgctaa tggcgtcgac       120

ggtgaatgga gctacgacga cgctaccaaa accttcacgg ttaccgaata a                171

<210>  24

<211>  171
<212>  DNA
<213>  Artificial Sequence

<220>
<223>  code DNA

<400>  24
accacctaca aactgatcgt gaaaggtaat acctttagcg gcgagactac gaccaaagcc      60

gttgatgctg aaacggccga gaaagcgttc aaacagtacg ctaacgagaa tggcgtctac     120

ggtgaatgga gctacgacga cgctaccaaa accttcacgg ttaccgaata a              171


<210>  25
<211>  171
<212>  DNA
<213>  Artificial Sequence

<220>
<223>  code DNA

<400>  25
accagctaca aactggtcat taaaggtgcg acctttagcg gcgagactgc gaccaaagcc      60

gttgatgctg ctgtggccga gcaaacgttc cgtgactacg ctaacaagaa tggcgtcgac     120

ggtgtatggg cctacgacgc cgctaccaaa accttcacgg ttaccgaata a              171


<210>  26
<211>  171
<212>  DNA
<213>  Artificial Sequence

<220>
<223>  code DNA

<400>  26
accacctacc gcctggtcat taaaggtgtg acctttagcg gcgagactgc gaccaaagcc      60

gttgatgctg ctacggccga gcaaacgttc cgtcagtacg ctaacgataa tggcatcacc     120

ggtgaatggg cctacgacac cgctaccaaa accttcacgg ttaccgaata a              171


<210>  27
<211>  171
<212>  DNA
<213>  Artificial Sequence

<220>
<223>  code DNA

<400>  27
accacctaca aactggtcgt gaaaggtaat accctgacag gctatactac gaccatcgcc      60

attgatactg ctacggccga gaaagagctc aaacagttcg ctaccgctaa taacgtcgac     120

ggtgaatgga gctacgacga cgctaccaaa accttcacgg ttaccgaata a              171

<210>  28
<211>  171
<212>  DNA
<213>  Artificial Sequence

<220>
<223>  code DNA

<400>  28
accacctaca aactgatcgt gaaaggtaat accctgacag gctatactac gaccatcgcc      60

gttgatgctg aaacggccga gaaagcgctc aaacagttcg ctaccgctaa taacgtcgac     120

ggtgaatgga gctacgacga cgctaccaaa accttcacgg ttaccgaata a              171


<210>  29
<211>  171
<212>  DNA
<213>  Artificial Sequence

<220>
<223>  code DNA

<400>  29
accacctaca aactgatcgt gaaaggtaat accctgacag gctatactac gaccatcgcc      60

attgatgctg ctacggccga gaaagagctc aaacagttcg ctaccgctaa tggcgtcgac     120

ggtgaatgga gctacgacga cgctaccaaa accttcacgg ttaccgaata a              171


<210>  30
<211>  171
<212>  DNA
<213>  Artificial Sequence

<220>
<223>  code DNA

<400>  30
accacctaca aactgatcgt gaaaggtaat accctgacag gctatactac gaccatcgcc      60

gttgatgctg aaacggccga gaaagcgctc aaacagttcg ctaacgagaa tggcgtctac     120

ggtgaatgga gctacgacga cgctaccaaa accttcacgg ttaccgaata a              171


<210>  31
<211>  171
<212>  DNA
<213>  Artificial Sequence

<220>
<223>  code DNA

<400>  31
accagctaca aactggtcat taaaggtgcg accctgacag gctatactgc gaccatcgcc      60

gttgatgctg ctgtggccga gcaaacgctc cgtgacttcg ctaacaagaa tggcgtcgac     120

ggtgtatggg cctacgacgc cgctaccaaa accttcacgg ttaccgaata a              171

<210>  32
<211>  171
<212>  DNA
<213>  Artificial Sequence

<220>
<223>  code DNA

<400>  32
accacctacc gcctggtcat taaaggtgtg accctgacag gctatactgc gaccatcgcc      60

gttgatgctg ctacggccga gcaaacgctc cgtcagttcg ctaacgataa tggcatcacc      120

ggtgaatggg cctacgacac cgctaccaaa accttcacgg ttaccgaata a               171


<210>  33
<211>  171
<212>  DNA
<213>  Artificial Sequence

<220>
<223>  code DNA

<400>  33
accacctaca aactgatcgt gaaaggtaat accctgagcg gctatactac gaccatcgcc      60

gttgatgctg aaacggccga gaaagcgctc aaacagttcg ctaccgctaa taacgtcgac      120

ggtgaatgga gctacgacga cgctaccaaa accttcacgg ttaccgaata a               171


<210>  34
<211>  171
<212>  DNA
<213>  Artificial Sequence

<220>
<223>  code DNA

<400>  34
accacctaca aactgatcgt gaaaggtaat accctgagcg gctatactac gaccatcgcc      60

attgatgctg ctacggccga gaaagagctc aaacagttcg ctaccgctaa tggcgtcgac      120

ggtgaatgga gctacgacga cgctaccaaa accttcacgg ttaccgaata a               171


<210>  35
<211>  171
<212>  DNA
<213>  Artificial Sequence

<220>
<223>  code DNA

<400>  35
accacctaca aactgatcgt gaaaggtaat accctgagcg gctatactac gaccatcgcc      60

gttgatgctg aaacggccga gaaagcgctc aaacagttcg ctaacgagaa tggcgtctac      120

ggtgaatgga gctacgacga cgctaccaaa accttcacgg ttaccgaata a               171

<210>  36
<211>  69
<212>  DNA
<213>  Artificial Sequence

<220>
<223>  oligo DNA

<400>  36
ctgggatcca ccacctacaa actggtcgtg aaaggtaata gctttaaagg cgagactacg        60

accaaagcc                                                                69


<210>  37
<211>  69
<212>  DNA
<213>  Artificial Sequence

<220>
<223>  oligo DNa

<400>  37
accaaagcca ttgatactgc tacggccgag aaagagttca aacagtacgc taccgctaat        60

aacgtcgac                                                                69


<210>  38
<211>  69
<212>  DNA
<213>  Artificial Sequence

<220>
<223>  oligo DNA

<400>  38
cgggaattct tattcggtaa ccgtgaaggt tttggtagcg tcgtcgtagc tccattcacc        60

gtcgacgtt                                                                69


<210>  39
<211>  69
<212>  DNA
<213>  Artificial Sequence

<220>
<223>  oligo DNA

<400>  39
ctgggatcca ccacctacaa actgatcgtg aaaggtaata cctttagcgg cgagactacg        60

accaaagcc                                                                69


<210>  40
<211>  69
<212>  DNA
<213>  Artificial Sequence

<220>
<223>  oligo DNA

<400>  40
accaaagccg ttgatgctga aacggccgag aaagcgttca aacagtacgc taccgctaat      60

aacgtcgac                                                              69


<210>  41
<211>  69
<212>  DNA
<213>  Artificial Sequence

<220>
<223>  oligo DNA

<400>  41
accaaagcca ttgatgctgc tacggccgag aaagagttca aacagtacgc taccgctaat      60

ggcgtcgac                                                              69


<210>  42
<211>  69
<212>  DNA
<213>  Artificial Sequence

<220>
<223>  oligo DNA

<400>  42
cgggaattct tattcggtaa ccgtgaaggt tttggtagcg tcgtcgtagc tccattcacc      60

gtcgacgcc                                                              69


<210>  43
<211>  69
<212>  DNA
<213>  Artificial Sequence

<220>
<223>  oligo DNA

<400>  43
accaaagccg ttgatgctga aacggccgag aaagcgttca aacagtacgc taacgagaat      60

ggcgtctac                                                              69


<210>  44
<211>  69
<212>  DNA
<213>  Artificial Sequence

<220>
<223>  oligo DNA

<400>  44
cgggaattct tattcggtaa ccgtgaaggt tttggtagcg tcgtcgtagc tccattcacc      60

gtagacgcc                                                              69

<210>  45
<211>  69
<212>  DNA
<213>  Artificial Sequence

<220>
<223>  oligo DNA

<400>  45
ctgggatcca ccagctacaa actggtcatt aaaggtgcga cctttagcgg cgagactgcg      60

accaaagcc                                                              69


<210>  46
<211>  69
<212>  DNA
<213>  Artificial Sequence

<220>
<223>  oligo DNA

<400>  46
accaaagccg ttgatgctgc tgtggccgag caaacgttcc gtgactacgc taacaagaat      60

ggcgtcgac                                                              69


<210>  47
<211>  69
<212>  DNA
<213>  Artificial Sequence

<220>
<223>  oligo DNA

<400>  47
cgggaattct tattcggtaa ccgtgaaggt tttggtagcg gcgtcgtagg cccatacacc      60

gtcgacgcc                                                              69


<210>  48
<211>  69
<212>  DNA
<213>  Artificial Sequence

<220>
<223>  oligo DNA

<400>  48
ctgggatcca ccacctaccg cctggtcatt aaaggtgtga cctttagcgg cgagactgcg      60

accaaagcc                                                              69


<210>  49
<211>  69
<212>  DNA
<213>  Artificial Sequence

<220>
<223>  oligo DNA

<400>  49
accaaagccg ttgatgctgc tacggccgag caaacgttcc gtcagtacgc taacgataat      60

ggcatcacc                                                              69


<210>  50
<211>  69
<212>  DNA
<213>  Artificial Sequence

<220>
<223>  oligo DNA

<400>  50
cgggaattct tattcggtaa ccgtgaaggt tttggtagcg gtgtcgtagg cccattcacc      60

ggtgatgcc                                                              69


<210>  51
<211>  69
<212>  DNA
<213>  Artificial Sequence

<220>
<223>  oligo DNA

<400>  51
ctgggatcca ccacctacaa actggtcgtg aaaggtaata ccctgacagg ctatactacg      60

accatcgcc                                                              69


<210>  52
<211>  69
<212>  DNA
<213>  Artificial Sequence

<220>
<223>  oligo DNA

<400>  52
accatcgcca ttgatactgc tacggccgag aaagagctca aacagttcgc taccgctaat      60

aacgtcgac                                                              69


<210>  53
<211>  69
<212>  DNA
<213>  Artificial Sequence

<220>
<223>  oligo DNA

<400>  53
ctgggatcca ccacctacaa actgatcgtg aaaggtaata ccctgacagg ctatactacg      60

accatcgcc                                                              69

<210>  54
<211>  69
<212>  DNA
<213>  Artificial Sequence

<220>
<223>  oligo DNA

<400>  54
accatcgccg ttgatgctga aacggccgag aaagcgctca aacagttcgc taccgctaat        60

aacgtcgac                                                                69


<210>  55
<211>  69
<212>  DNA
<213>  Artificial Sequence

<220>
<223>  oligo DNA

<400>  55
accatcgcca ttgatgctgc tacggccgag aaagagctca aacagttcgc taccgctaat        60

ggcgtcgac                                                                69


<210>  56
<211>  69
<212>  DNA
<213>  Artificial Sequence

<220>
<223>  oligo DNA

<400>  56
accatcgccg ttgatgctga aacggccgag aaagcgctca aacagttcgc taacgagaat        60

ggcgtctac                                                                69


<210>  57
<211>  69
<212>  DNA
<213>  Artificial Sequence

<220>
<223>  oligo DNA

<400>  57
ctgggatcca ccagctacaa actggtcatt aaaggtgcga ccctgacagg ctatactgcg        60

accatcgcc                                                                69


<210>  58
<211>  69
<212>  DNA
<213>  Artificial Sequence

<220>
<223>  oligo DNA

<400>  58
accatcgccg ttgatgctgc tgtggccgag caaacgctcc gtgacttcgc taacaagaat      60

ggcgtcgac                                                              69


<210>  59
<211>  69
<212>  DNA
<213>  Artificial Sequence

<220>
<223>  oligo DNA

<400>  59
ctgggatcca ccacctaccg cctggtcatt aaaggtgtga ccctgacagg ctatactgcg      60

accatcgcc                                                              69


<210>  60
<211>  69
<212>  DNA
<213>  Artificial Sequence

<220>
<223>  oligo DNA

<400>  60
accatcgccg ttgatgctgc tacggccgag caaacgctcc gtcagttcgc taacgataat      60

ggcatcacc                                                              69


<210>  61
<211>  69
<212>  DNA
<213>  Artificial Sequence

<220>
<223>  oligo DNA

<400>  61
ctgggatcca ccacctacaa actgatcgtg aaaggtaata ccctgagcgg ctatactacg      60

accatcgcc                                                              69


<210>  62
<211>  171
<212>  DNA
<213>  Artificial Sequence

<220>
<223>  code DNA

<400>  62
accacctaca aactgatcct gaacggtaag accctgaaag gcgagactac gaccgaagcc      60

gttgatgctg ctacggccga gaaagtgttc aaacagtacg ctaacgataa tggcgtcgac     120

ggtgaatgga cctacgacga cgctaccaaa accttcacgg ttaccgaata a 171

<210> 63
<211> 171
<212> DNA
<213> Artificial Sequence

<220>
<223> code DNA

<400> 63
accacctaca aactggtcat caacggtaag accctgaaag gcgagactac gaccgaagcc 60

gttgatgctg ctacggccga gaaagtgttc aaacagtacg ctaacgataa tggcgtcgac 120

ggtgaatgga cctacgacga cgctaccaaa accttcacgg ttaccgaata a 171

<210> 64
<211> 171
<212> DNA
<213> Artificial Sequence

<220>
<223> code DNA

<400> 64
accacctaca aactggtcat caacggtaag accctgaaag gcgagactac gaccaaagcc 60

gttgatgctg agacggccga gaaagcgttc aaacagtacg ctaacgataa tggcgtcgac 120

ggtgtatgga cctacgacga cgctaccaaa accttcacgg ttaccgaata a 171

<210> 65
<211> 171
<212> DNA
<213> Artificial Sequence

<220>
<223> code DNA

<400> 65
accacctaca aactggtcat caacggtaag accctgacag gctatactac gaccatcgcc 60

gttgatgctg agacggccga gaaagcgctc aaacagttcg ctaacgataa tggcgtcgac 120

ggtgtatgga cctacgacga cgctaccaaa accttcacgg ttaccgaata a 171

<210> 66
<211> 69
<212> DNA
<213> Artificial Sequence

<220>
<223> oligo DNA

<400> 66
ctgggatcca ccacctacaa actgatcctg aacggtaaga ccctgaaagg cgagactacg 60

accgaagcc                                                          69

<210>  67
<211>  69
<212>  DNA
<213>  Artificial Sequence

<220>
<223>  oligo DNA

<400>  67
accgaagccg ttgatgctgc tacggccgag aaagtgttca aacagtacgc taacgataat      60

ggcgtcgac                                                          69

<210>  68
<211>  69
<212>  DNA
<213>  Artificial Sequence

<220>
<223>  oligo DNA

<400>  68
cgggaattct tattcggtaa ccgtgaaggt tttggtagcg tcgtcgtagg tccattcacc      60

gtcgacgcc                                                          69

<210>  69
<211>  69
<212>  DNA
<213>  Artificial Sequence

<220>
<223>  oligo DNA

<400>  69
ctgggatcca ccacctacaa actggtcatc aacggtaaga ccctgaaagg cgagactacg      60

accgaagcc                                                          69

<210>  70
<211>  69
<212>  DNA
<213>  Artificial Sequence

<220>
<223>  oligo DNA

<400>  70
ctgggatcca ccacctacaa actggtcatc aacggtaaga ccctgaaagg cgagactacg      60

accaaagcc                                                          69

<210>  71
<211>  69
<212>  DNA
<213>  Artificial Sequence

<220>
<223>  oligo DNA

<400>  71
accaaagccg ttgatgctga gacggccgag aaagcgttca aacagtacgc taacgataat      60

ggcgtcgac                                                              69


<210>  72
<211>  69
<212>  DNA
<213>  Artificial Sequence

<220>
<223>  oligo DNA

<400>  72
cgggaattct tattcggtaa ccgtgaaggt tttggtagcg tcgtcgtagg tccatacacc      60

gtcgacgcc                                                              69


<210>  73
<211>  69
<212>  DNA
<213>  Artificial Sequence

<220>
<223>  oligo DNA

<400>  73
ctgggatcca ccacctacaa actggtcatc aacggtaaga ccctgacagg ctatactacg      60

accatcgcc                                                              69


<210>  74
<211>  69
<212>  DNA
<213>  Artificial Sequence

<220>
<223>  oligo DNA

<400>  74
accatcgccg ttgatgctga gacggccgag aaagcgctca aacagttcgc taacgataat      60

ggcgtcgac                                                              69